# EUROPEAN PATENT APPLICATION

(11) **EP 3 695 876 A1**
(43) Date of publication of application: **19.08.2020**
(21) Application number: 19157611.5
(22) Date of filing: 18.02.2019
(51) Int. Cl.: A61M 37/00

(54) **PENCIL FOR INSERTING MICRO-IMPLANTS INSIDE UPPER SKIN LAYERS**

(71) Applicant: L'OREAL, 75008 Paris (FR)
(72) Inventor: BORDEAUX, Dominique, 94152 CHEVILLY LA RUE (FR)

(57) **Abstract**

The invention relates to an injection pencil (10) extending along a longitudinal axis (X) for injecting at least one material into human keratinous materials, comprising at least a hollow body (2) intended to house at one end, an injection head (22) in the form of a tip pencil for the delivery of a micro-implant (40) of the at least one material and at the other end, at least one control member (9) acting on the said micro-implant (40) and moving the said micro-implant (40) from an inactive position in which it is arranged inside the said body (2) to an active position in which it is at least partially arranged outside the said body (2).

## Description

The present invention relates to devices for injecting at least one material in keratinous materials such human skin and mucous membranes.

### Background of the invention

US 8167852 and US 8834423 disclose devices that comprise an array of micro-implants (also called "microneedles") carried by a support.

Such devices may be useful for delivery of cosmetic materials, drugs or vaccines into the skin. An advantage is that they can penetrate the stratum corneum without the pain caused by conventional needles and can be self-administered.

The production of patches comprising solid microneedles arrays on a backplate has been described in various applications such as WO2008/139786, WO2009/040548, WO2015/147040 and WO2016/076442.

Theoretically the microneedles require a very low pressure to be inserted into skin, potentially allowing arrays with hundreds of such microneedles to be inserted into skin by hand without any aid.

At the time of use, the microneedles arrays is pressed into the skin, and the micro-implants penetrate in the stratum corneun, epidermis or dermis. It is difficult to press evenly the micro-implants due to suppleness of the skin and there may remain a portion of length of the micro-implants that is not inserted into the stratum corneum, epidermis or dermis. When the backplate is removed, there may remain onto the backplate some portion of the micro-implants, which is thus lost.

Furthermore, the micro-implants may not remain inserted into the keratinous materials due to skin motions and the capability to recover its initial shape after pressure on the device is done. A counter pressure occurs expelling the backplate away thus withdrawing the micro-implants out of the skin. Backplate's rigidity is a reason for micro-implants being rejected before during skin motions. Some part of the micro-implants which is not inserted cannot dissolve or disintegrate in the keratinous materials and is lost. The quantity of material delivered into skin may thus be relatively low and expected efficacy very weak. Morever regular patches are note able to focus on specific zone such like skin depression called "wrinkle" due to their oversized surface.

### Summary of the invention

There is a need for improving further devices for injecting a material into keratinous materials and overcoming at least some of the drawbacks of the prior art devices. It would be beneficial to improve delivery and to reduce wastes.

In particular , there is a strong need to propose solutions to help microneedles :
- Puncturing skin entirely and evenly,
- Remaining into the skin,
- Targeting the accurate zone and depth,
- Delivering the full compounds and the right quantity of it to reach the expected efficacy.

Exemplary embodiments of the invention relate to an injection pencil extending along a longitudinal axis (X) for injecting at least one material into human keratinous materials, the device comprises at least a hollow body intended to house at its first end, an injection head in the form of a tip pen for the delivery of a micro-implant of the at least one material and at its second end, at least one control member acting on the said micro-implant via at least one conversion member converting a movement of the said control member into a translational movement of the said micro-implant and moving the said micro-implant from an inactive position in which it is arranged inside the said body to an active position in which it is at least partially arranged outside the said body.

The injection device according to the invention is a specific delivery tool that enables to drive micro-implants precisely inside skin. The invention device is also able to hold micro-implants made up with bio polymers.

Another advantage of the device according to the invention is that the micro-implants can be manufactured according to a wide range of processes, such as 3D-printing, regular molding and casting, compression but also extrusion process.

In one embodiment, the device is able to diagnose the skin before bio implants transfer into the skin and to adjust its parameters to provide proper pressure or energy to optimize implants transfer into human tissues, such as skin, lips, scalp, gums.

The device according to the invention can also enhance bioavailability of active compounds thanks to higher insertion amount and full implant insertion which delivers actives upon time.

Furthermore, the invention makes it easier to inject the entire micro-implants in the body, with no loss of material. Wastes are reduced and delivery is increased.

The absence of a conventional backplate helps penetration of the micro-implants into the stratum corneum, dermis or epidermis and induce a better delivery of the material to be injected, even in skin regions of high suppleness.

The invention enables to use micro-implants having shapes that could not be unmolded, such as shapes with cutbacks. The invention also enables shapes that would brake during a conventional de-molding process. The invention may enable the make a micro-implant using a combination of different materials that would render the micro-implant difficult to unmold.

The invention also helps to associate micro-implants of different materials in a same treatment unit.

The device according to the invention may be used for any cosmetic treatment of the skin, such as wrinkles filling, spots treatment and coloring of the skin.

The wording "micro-implant" includes micro-needles and micro-structures.

The pencil may be pressed against the body before injection. The pencil may have for this purpose an application surface for contacting the keratinous materials when the at least one micro-implant is expelled out of the pencil.

### Micro-implant

A micro-implant may have a largest transverse dimension at its base no greater than 1500 microns, for example a largest transverse dimension ranging from 1 to 1500 microns. The section of a micro-implant is preferably circular, in which case the largest diameter of a micro-implant may range from 1 to 1000 microns, for example between 200 and 400 microns.

A length of the micro-implant may range from 100 to 1500 microns, better from 200 to 800 microns, even better from 400 to 600 microns.

The micro-implant may comprise a proximal cylindrical portion of constant section and a distal portion having a section that narrows toward the tip of the micro-implant, for example a conical distal portion.

The proximal and distal portions may be of substantially same length, for example lₚ * 0,8 < l_{d} < lₚ*1.2, where lₚ is the length of the proximal portion and l_{d} is the length of the distal portion.

The length of the proximal portion may range from 200 to 400 micron and the length of the distal portion may range from 200 to 400 microns also. Other ranges are possible, of course.

The pencil may be discarded after first use or after micro-implants have been expelled therefrom. The pencil can be re-usable to generate new micro implants.

All the micro-implants may be made of a same material. In a variant, some of the micro-implants are made of a first material and some others are made of at least one second material different from the first one. A micro-implant may be made of different materials; for example, a proximal portion of the micro-implant is made in a first material and a distal portion of the micro-implant is made of a second material different from the first one.

The at least one micro-implant may comprise at least one anchoring relief for anchoring the micro-implant into the keratinous materials. The at least one micro-implant may comprise at least one of a serrated or harpooning head. The invention allows formation of cutbacks in the micro-implants.

The at least one micro-implant may comprise at least one material selected among implantable substances such an implantable class of hyaluronic acid (HA), PVP, PEG or xylitol. The material of the micro-implants may be pure HA or be based on HA and xylitol. These materials are given as examples.

The at least one micro-implant may carry actives that may be cosmetic actives, or drugs or vaccines. The micro-implant may be made of a material that is resorbable, preferably water-dissolvable or soluble in any body fluid.

The material of the micro-implants may be of high swell ability and high viscoelasticity.

The pencil may comprise at least two micro-implants of different materials, volumes, lengths and/or shapes.

The pencil may comprise a liquid in contact with the at least one micro-implant. This liquid may be in contact with the micro-implant in the packaging of the unit, or the liquid may be in contact just before or at the time of use, as a result of an action from the user. For example, the liquid is encapsulated and released when the micro-implants are expelled or just prior to the ejection.

### Preferred embodiments

According to further advantageous aspects of the invention, the device comprises one or several of the following features taken in isolation or in any technically possible combinations:
- It comprises a micro-implant guide comprising a conduit for the passage of the micro-implant and for its guidance in translational movement along the axis X. The movement of the lead of micro-implant is straight and safe.
- It comprises an elastic member designed to store mechanical energy and cinematically connected to the said micro-implant, the control member allowing the said elastic member to at least partially release the said mechanical energy, in response to an action of a user to move the micro-implant from the inactive position to the active position. The movement of the lead is more flexible.
- It comprises a retractable retaining member designed to retain the said micro-implant in its active position. The movement of the lead is more flexible, creating a pleasant sensation for the user.
- It comprises a micro-implant brake connected to the injection head. The micro-implant stops precisely.
- It comprises a transparent window, or a transparent cylindrical portion, through which the kinematics corresponding to the movement of the said micro-implant from its inactive position to its active position can be observed. The user might be aware of the course of the lead of micro-implant.
- A micro-implant feeding mechanism comprising a receiving portion to hold the micro-implant disposed in an inside portion of the body. A reserve of micro-implants may be formed. The storage is hygienic.
- It comprises a cutting mechanism designed to cut the micro-implants at a predetermined length before being expulsed from the body, the cutting mechanism comprising notably a blade, a plier or a clip. The micro-implant is sharpened. It penetrates more easily into the skin.
- The injection head comprises a rotation member or a translation member for adjusting the length of the micro-implant. The handling is easy.
- It comprises an energy generating member, preferably a heating member, a cooling member or a lighting member. Such a member may enhance the penetration of the micro-implant into the skin.
- It comprises a reservoir for storing a liquid, the liquid and the material of the micro-implants being selected so that the liquid dissolves the material or react to generate another material. The generated material might bring special effects on the skin of the consumer.
- The micro-implants are made of a first material and some others are made of at least one second material different from the first one. The effect of this mixture might be of interest for obtaining some effects.
- It comprises an image sensor and a processor for automated detection of a target zone of the keratinous material and for signaling a user when the system is properly positioned relative to the target zone prior to expelling the at least one micro-implant and/or for automated triggering of the injection of the at least one micro-implant when the system is properly positioned relative to the target zone. Such a system might be easier for the user.
- It is a propelling pencil. The ergonomics and the efficiency are increased.

The injection pencil may comprise an energy generator, e.g. a motor or a spring, a compressed gas capsule for displacing the injection pencil relative to a unit or vice versa, the injection pencil and the unit forming a system.

The injection pencil may be configured for bringing into contact a liquid with the at least one micro-implant. The bringing into contact may occur prior to expelling the at least one micro-implant.

The bringing into contact may occur after the at least one micro-implant has been injected into the keratinous materials.

The injection pencil may comprise a reservoir for storing said liquid.

The liquid and the material of the micro-implants may be selected so that the liquid dissolves the material or react to generate another material. The liquid may also cause the material of the micro-implant to swell and or to generate gaz.

The injection pencil may be configured for extemporaneously bringing said liquid into contact with the micro-implants.

The injection pencil may comprise an image sensor and a processor for automated detection of a target zone of the keratinous material and for signaling a user when the system is properly positioned relative to the target zone prior to expelling the at least one micro-implant and/or for automated triggering of the injection of the at least one micro-implant when the system is properly positioned relative to the target zone. The image sensor may be an optical sensor such as a camera or a non-optical sensor such as a capacitive sensor.

The injection pencil may be connected (wireless, wifi, Bluetooth, etc..) to a smartphone, computer or a diagnosis device.

The target zone may comprise a wrinkle or any skin depression.

When the image sensor is off-centered relative to the micro-implants, the injection pencil is configured for detecting the displacement of the injection system along the skin to compensate this off-centering before triggering the injection. This displacement may be detected optically.

The system may comprise an electromechanical actuator for vibrating the injection pencil. This may help to create a massaging action against pain and/or improving the diffusion of the material within the stratum corneum, epidermis or dermis.

The injection pencil may comprise a cold source for lowering the temperature of the unit and/or of the keratinous materials prior to, during or after the injection. This may help reduce inflammatory side effects, pain and/or make the dermis or epidermis firmer.

The injection pencil may comprise a heat source for increasing the temperature of the unit and/or the keratinous materials prior to, during or after the injection. This may lower the rigidity of the material of the core and help micro-implants to be expelled therefrom.

The injection pencil may comprise a light source for projecting light onto a target zone where the at least one micro-implant is to be injected. This may help the user to position the system in the area to be treated and/or facilitate detection of wrinkles or other element to treat with the system.

The system may comprise a storage compartment for storing a plurality of the treatment units. Such a storage compartment may be a cartridge in which a set of units are present. The user may insert the cartridge into the injection device and replace it by a new one after all units of the cartridge have been used.

The injection device may be configured for micro-implants of different volumes, materials and/or length or shapes.

The pencil may comprise a feeding mechanism configured for automatic replacement of a used micro-implant by a fresh micro-implant coming from the storage compartment.

The injection pencil may be configured for automatic identification of a micro-implant. The injection pencil may operate under different conditions depending the injection pencil that has been identified.

### Non-therapeutic method

A further object of the invention is a non-therapeutic method for treating keratinous materials, comprising injecting at least one micro-implant into the keratinous materials using the pencil as defined above. Preferably, the at least one micro-implant is integrally expelled from the pencil during the injection.

The method may be performed for treating wrinkles.

### Brief description of the drawings

The invention will be better understood in view of the following description of non-limitative embodiments and in view of the appended drawings in which:
Fig. 1 is a schematic perspective front view of an injection pencil according to the invention,
Figs. 2 is a schematic perspective front view of the control member of the pencil of Fig. 1.
Figs. 3 is a schematic perspective front view of the micro-implant guide of the pencil of Fig. 1.
Figs. 4 is a schematic perspective front view of the injection head of the pencil of Fig. 1.
Fig. 5 is a partial and schematic view in longitudinal section of an injection pencil according to an exemplary embodiment of the invention, before an injection,
Fig. 6 is a partial and schematic view in longitudinal section of an injection pencil according to Fig. 5, during an injection,
Fig. 7 is a partial and schematic view in longitudinal section of an injection pencil according to an exemplary embodiment of the invention, after an injection,
Fig.8 is a partial and schematic view in longitudinal section of the injection head of Fig. 5.

### Detailed description of the drawings

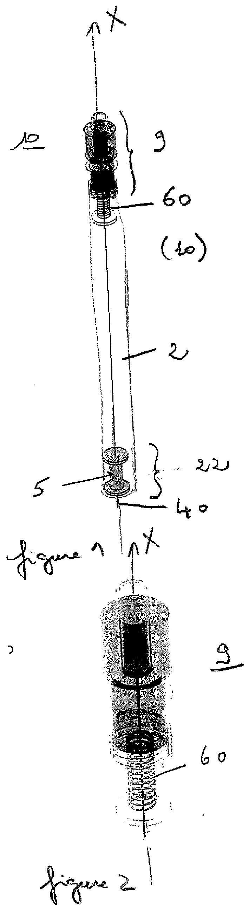

An example of an injection pencil 10 according to the embodiment of the present invention is shown in FIG. 1. It has a cylindrical body 2 extending longitudinally along an axis X. This body 2 has a forward end and a rear end. An injection head 22 is mounted at the forward end. It comprises a micro-implant guide 5 with a conduit 6 designed to guide the micro-implant 40 translationally along the axis X and to protect it as it emerges from the injection head. A control member 9 is mounted at the rear end for moving the micro- implant from a rest position to an active position.

The body mainly serves to accommodate micro-implants in the rest configuration and is dimensioned to give the body a conventional length.

The injection head 22 is intended to contain an extraction control mechanism of the micro-implant 40 out of the body 2.

The device 10 might deliver a whole micro-implant or just a part of it, which is sharpen and cut at will. It might also be able to hold liquid(s) inside the control member 9 that can be delivered at the same time than the micro-implant at the targeted site and depth of skin.

To use the system depicted in Fig. 1, the user can hold the body 22 with one hand and positions a finger on the control member 9. Then, he pushes the finger in the direction of the injection head. The pencil 10 is hold above a zone of the body where the micro-implants 40 should be injected.

The user triggers the control member 9 and the driving mechanism through a conversion member, causes the micro-implants 40 to move toward the zone and to be expelled out of the injection head 22.

FIG.2 represents the control member 9, here having the form of a bolt mounted mobile in rotation between two extreme positions to release the micro-implant, in the use configuration, and allow its return to inside the hollow body 2.

The control member 9 could also be mobile in translation.

The control member 9 is able to rotate precisely in order to adjust, when activated, the depth insertion of micro implant into the skin.

The internal volume of the control member 9 can be directly connected to the guide 5 to deliver fluids at will. The elastic member 60, as a spring, can produce force for micro-implant insertion into skin. The injection can be adjusted according to information collected by a sensor located inside the injection head 22 of the device 10.

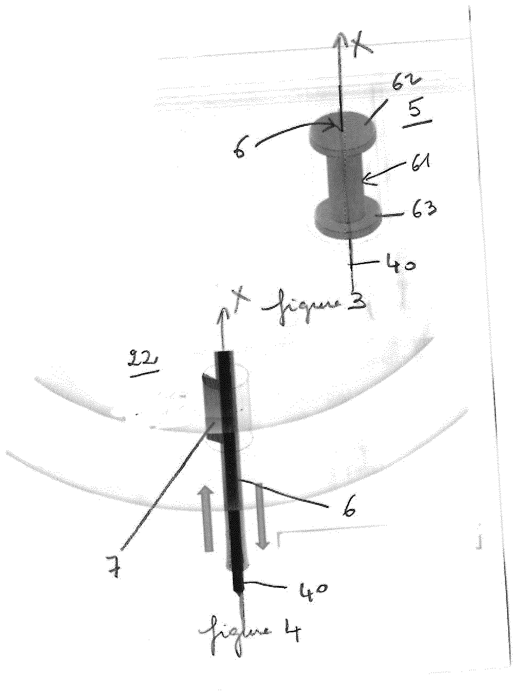

The guide 5 represented FIG.3 comprises a conduit 6 to drive the micro-implant toward the injection head 22. On the represented example it has the shape of a coil with a cylindrical middle portion 61 and disc portions 62, 63 at each ends. The guide could have any other structure, but preferably the conduit 6 has the same longitudinal axis as the pencil 10.

The delivery head 22 represented on FIG. 3 leads the canal 6 to the outside of the pencil 10, to deliver the micro-implant 40. It might comprise a cutting mechanism 7 and can hold different energies generator to produce heat, micro currents, vibrations, light for example. It can also display light target to locate insertion point at the skin surface.

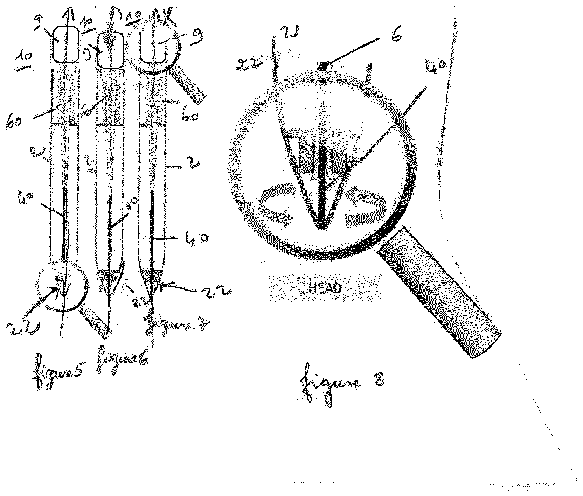

FIG.5 shows the device 10 before the injection as the micro-implant 40 is inside the injection head 22, housed in the conduit 6.

FIG.6 shows the device 10 during the injection of the micro-implant 40 as it is coming out of the ejection head into the skin of the person, when the consumer presses the control member in the direction of the arrow.

FIG.7 shows the micro-implant 40 dragged along the longitudinal axis X in the direction of the control member 9 after use. In position of storing represented FIG. 8, the micro-implant is stored into the injection head.

The invention is not limited to the examples represented on the figures. The invention deals with any kind of pencil to deliver a micro-implant.

## Claims

1. An injection pencil (10) extending along a longitudinal axis (X) for injecting at least one material into human keratinous materials, **characterized by** the fact that it comprises at least a hollow body (2) intended to house at one end, an injection head (22) in the form of a tip pencil for the delivery of a micro-implant (40) of the at least one material and at the other end, at least one control member (9) acting on the said micro-implant (40) via at least one conversion member converting a movement of the said control member (9) into a translational movement of the said micro-implant (40) and moving the said micro-implant (40) from an inactive position in which it is arranged inside the said body (2) to an active position in which it is at least partially arranged outside the said body (2).

2. An injection pencil (10) according to claim 1, **characterized by** the fact that it comprises a micro-implant guide (5) comprising a conduit (6) for the passage of the micro-implant (40) and for its guidance in translational movement along the axis (X).

3. An injection pencil according to one of the preceding claims, **characterized by** the fact that it comprises an elastic member (60) designed to store mechanical energy and cinematically connected to the said micro-implant, the control member (9) allowing the said elastic member (60) to at least partially release the said mechanical energy, in response to an action of a user to move the micro-implant (40) from the inactive position to the active position.

4. An injection pencil according to one of the preceding claims, **characterized by** the fact that it comprises a retractable retaining member designed to retain the said micro-implant (40) in its active position.

5. An injection pencil according to one of the preceding claims, **characterized by** the fact that it comprises a micro-implant brake connected to the injection head (22).

6. An injection pencil according to one of the preceding claims, **characterized by** the fact that it comprises a transparent window, or a transparent cylindrical portion, through which the kinematics corresponding to the movement of the said micro-implant (40) from its inactive position to its active position can be observed.

7. An injection pencil according to one of the preceding claims, **characterized by** the fact that a micro-implant feeding mechanism comprising a receiving portion to hold the micro-implant (40) disposed in an inside portion of the body (2).

8. An injection pencil according to one of the preceding claims, **characterized by** the fact that it comprises a cutting mechanism (7) designed to cut the micro-implants at a predetermined length before being expulsed from the body (2), the cutting mechanism (7) comprising notably a blade, a plier or a clip.

9. An injection pencil according to one of the preceding claims, **characterized by** the fact that the injection head (22) comprises a rotation member or a translation member for adjusting the length of the micro-implant (40).

10. An injection pencil according to one of the preceding claims, **characterized by** the fact that it comprises an energy generating member, preferably a heating member, a cooling member or a lighting member.

11. An injection pencil according to one of the preceding claims, **characterized by** the fact that it comprises a reservoir for storing a liquid, the liquid and the material of the micro-implants being selected so that the liquid dissolves the material or react to generate another material.

12. An injection pencil according to one of the preceding claims, **characterized by** the fact that the micro-implants (40) are made of a first material and some others are made of at least one second material different from the first one.

13. The injection pencil according to one of the preceding claims, **characterized by** the fact that it comprises an image sensor and a processor for automated detection of a target zone of the keratinous material and for signaling a user when the system is properly positioned relative to the target zone prior to expelling the at least one micro-implant and/or for automated triggering of the injection of the at least one micro-implant when the system is properly positioned relative to the target zone.

14. An injection pencil according to one of the preceding claims, **characterized by** the fact that it is a propelling pencil.
